# EUROPEAN PATENT SPECIFICATION

(11) **EP 1 644 051 B1**
(45) Date of publication and mention of the grant of the patent: **02.01.2008**
(21) Application number: 04743535.9
(22) Date of filing: 23.07.2004
(51) Int. Cl.: A61L 2/18, A61L 2/26, A61L 2/00, C01B 11/02, C11D 3/00

(54) **CHLORINE DIOXIDE GENERATION**
ERZEUGUNG VON CHLORDIOXID
PRODUCTION DE DIOXYDE DE CHLORE

(30) Priority: 23.07.2003 GB 0317155
(43) Date of publication of application: 12.04.2006
(73) Proprietor: Tristel PLC, Fordham Road, Snailwell Newmarket CB8 7NY (GB)
(72) Inventor: HAWKER, Michael, John, Hadleigh IP7 5AP (GB); ALLEN, Timothy Derek, Poole BH17 0GD (GB); SWINNEY, Paul, Snailwell, Newmarket CB8 7NY (GB)
(74) Representative: Gemmell, Peter Alan
(86) International application number: PCT/GB2004/003203
(87) International publication number: WO 2005/011759

(56) References cited:
- EP-A- 1 310 263
- WO-A-03/000586
- US-A- 4 534 952
- US-A- 6 007 772
- US-A- 6 051 135

## Description

### FIELD OF THE INVENTION

The present invention relates to a method of producing chlorine dioxide on demand, and to a chlorine dioxide generator for use in the method. The invention is particularly for use in a medical facility for producing a stream of aqueous chlorine dioxide for sterilizing medical and dental equipment or for wound irrigation or skin asepsis.

### BACKGROUND TO THE INVENTION

Chlorine dioxide is an important sterilizing agent in medical facilities such as surgeries and hospitals. It may be formed from mixtures of various reagents including: chlorite and acid; chlorate, peroxide and acid; and chlorite, hypochlorite, and a suitable buffer. Chlorine dioxide has excellent sterilizing and bactericidal properties, and oral ingestion in man and animals has been shown to be relatively safe.

The cleaning of endoscopes and other medical equipment with suitable chlorine dioxide solutions is described in European Patent Number 0 785 719 and United States Patent Numbers 5,696,046 and 6,007,772.

It is not always convenient to mix up batches of chlorine dioxide solutions for use in sterilizing equipment or other applications. Accordingly, there is a need for apparatus to produce chlorine dioxide 'on demand' in a medical environment.

Apparatus for producing chlorine dioxide is known from US patent numbers 4,534,952 and 6,051,135, and International patent publication number WO 03/000586. However, these apparatuses are intended for applications such as pulp bleaching, fat bleaching, and water treatment. In many of these applications, reagents such as sulphuric acid may be tolerated, as may the presence of unreacted reagents and side-products such as foam or froth in the chlorine dioxide solution.

### SUMMARY OF THE INVENTION

According to a first aspect of the present invention there is provided a method of producing a stream of aqueous chlorine dioxide sterilizing fluid suitable for use in sterilizing medical and dental equipment or for wound irrigation or skin asepsis, the method comprising:
pumping fluid portions of a first reagent and a second reagent into the proximal end of an elongate reaction chamber, the reagents being selected to react together to form aqueous chlorine dioxide;
providing a stream of water around or adjacent to the distal end of the reaction chamber;
continuing to pump fluid portions of said reagents so as to cause the reagent mixture to travel through the reaction chamber and then into the stream of water to form a stream of aqueous chlorine dioxide sterilizing fluid;
wherein the pumping rates and the internal dimensions of the elongate reaction chamber are selected so that the reaction to form chlorine dioxide is substantially complete when the reagent mixture exits the reaction chamber; and
continuing pumping fluid portions of said reagents until a desired quantity of said sterilizing fluid has been produced;
wherein the pumping of each reagent is carried out by means of a diaphragm pump or a piston pump via a pressure-control valve so that each fluid portion is pumped at substantially constant pressure.

By pumping through a pressure-control valve, accurately metered portions of each reagent may be dispensed. This in turn means that an accurately metered quantity of chlorine dioxide can be injected into the water stream to provide a sterilizing fluid having a chlorine dioxide concentration which is appropriate to the specific application for which it is required.

The stream of water may be mains-pressure water or may be pumped at any other pressure appropriate to the intended application. For example, the chlorine dioxide may be injected into water pumped at low pressure from a bottled supply for point of use applications such as wound irrigation during surgery.

The method enables the provision of chlorine dioxide on a continuous basis for direct injection into a water supply at a rate dictated by the application. Only two separate pumps are needed when mains water is used, or three pumps for applications requiring the use of bottled water.

The fluid portions may be pumped simultaneously or sequentially. It is preferred that the fluid portions are pumped simultaneously. In one embodiment the fluid portions are pumped alternately at low flow rates, and simultaneously at higher flow rates. This arrangement facilitates adjustment to a variety of flow rates within the performance specifications of the pumps. For very low flow rates, fractional volumes may be pumped, compared to the volume dispensed on a complete pump cycle. It is preferred that each reagent is pumped at the same rate, so that equal volumes are mixed.

It is important that the reagents are selected such that the sterilizing fluid is physiologically acceptable for applications such as wound irrigation. Preferred reagents are chlorite (notably sodium chlorite) and organic acids (notably a mixture of citric acid, sorbic acid and boric acid). For convenience hereinafter, the invention will be described with reference to the use of sodium chlorite solution as the first reagent (or 'base'), and a mixture of solutions of citric, sorbic and boric acids as the second reagent (or 'activator'). However, it is to be understood that the invention is not limited to this preferred embodiment. For applications involving sterilization of medical apparatus, a corrosion inhibitor may be included, notably in the activator. Suitable corrosion inhibitors are described in EP 0 785 719. These inhibitors are preferred for the present application, but other suitable corrosion inhibitors may be employed.

We have found that either piston pumps or diaphragm pumps are suitable for use in the present invention. The pump body and valve components coming into contact with ClO₂ are preferably made from stainless steel, ceramics, or a chemically resistant grade of plastics material such as PVDF or PTFE. Diaphragm pumps are particularly preferred for cost considerations, and the invention will for convenience be described herein with reference to the use of diaphragm pumps. The diaphragms are preferably coated with PTFE.

Each pump is preferably provided with a vent valve to allow it to be primed. In a preferred embodiment, the pumps are electronically controlled using a stepper motor. It is particularly preferred that the stepper motor shaft is provided with an optical encoder for precise measurement of the shaft movement. By feeding measurement data from the optical encoders to the electronic controller, very precise measurement and control of the pump operation can be achieved. This in turn allows precisely controlled quantities of aqueous chlorine dioxide to be fed to the water stream.

In addition to controlling the pumps precisely, it is also desirable to measure the concentration of chlorine dioxide in the sterilizing fluid, for example by using an ion selective electrode or other probe to confirm that this is within acceptable limits or to trigger adjustments in the process parameters. It is preferred also to measure the water flow rate and to operate the method under a closed loop feedback control. Depending on the desired chlorine dioxide concentration in the sterilizing fluid, and the flow rate, the pumps are controlled to meter the reagents to the reaction chamber at the required rate, which is monitored by means of the optical encoders and dynamically adjusted if necessary.

The probe may be linked to a processor which gives a visible or audible signal when the measured concentration of chlorine dioxide is within specified limits. For example, white, green and red lights could indicate respectively: too low a concentration, an acceptable concentration, and too high a concentration.

Because of space limitations in medical environments such as operating theatres it is preferred that the reaction chamber is curved or bent so as to make it more compact. It is particularly preferred that the reaction chamber is a coil, notably a helical coil. The invention will, for convenience, be described with reference to the use of a helical coil. A preferred dwell time in the coil is in the range 20-40 seconds, notably about 30 seconds. We have found that for pump rates to achieve 20 ppm of ClO₂ in water, at a flow rate of 20 l/min, a 20 ml capacity mixing coil with an internal diameter of about 4 mm is appropriate, while for larger mixing coils of 100 ml or 200 ml capacity (for achieving 100 ppm or 200 ppm respectively) an internal diameter of about 8 mm works well. In each case, the coil capacity is selected so that a dwell time of about 30 seconds will produce the desired ClO₂ concentration in the sterilizing fluid.

Vent valves may be provided to permit purging of reagents and chlorine dioxide from the reaction chamber. By purging the reaction chamber when chlorine dioxide is not required, the life of the reaction chamber, and associated valve members, may be prolonged. In a preferred embodiment, the valve arrangement permits flushing of the reaction chamber with water from the water supply that provides the water stream to form the sterilizing fluid.

### Product variants

The ClO₂ generator must be capable of providing ClO₂ for a variety of dosing requirements. Some will require ClO₂ to be injected into a mains water supply at up to 400 kPa, others will require ClO₂ to be injected into water pumped at low pressure from a bottled supply for point of use applications such as wound irrigation during surgery. The concentration of ClO₂ and flow rate will vary depending upon the application. Typically ClO₂ concentrations will be in the range 5 ppm to 200 ppm, notably 20 ppm to 200 ppm, and flow rates of dosed water in the range 0 to 30 litres per minute,

**Product variant 1 -** A device that adds ClO₂ to the incoming mains water supply at a concentration of 20 ppm for use in the rinse cycle of endoscope washer disinfection machines.

**Product variant 2 -** A device that adds ClO₂ to the incoming mains water supply at a concentration of 20 ppm for use in the rinse cycle of endoscope washer disinfection machines and adds ClO₂ to the incoming mains water supply at a concentration of 80 ppm for use in the decontamination cycle.

**Product variant 3 -** A device that adds ClO₂ to the incoming mains water supply at a concentration of 200 ppm for use in the decontamination cycle of endoscope washer disinfection machines.

**Product variant 4 -** A device that automatically adds ClO₂ at a dilution of 20 ppm into the 'break' tanks that are fitted into the mains water supply circuit at multiple locations in hospitals. Break tanks are installed to ensure that there is no back contamination from the hospital point of use into the incoming mains water supply. ClO₂ at a dilution of 20 ppm is currently manually dropped into the tank by the maintenance team. This activity could be automated.

**Product variant 5 -** A device particularly for use in the Endoscope cleaning room of hospitals which have a washing regime that uses 4 sinks. Sink 1 is for detergent. Sink 2 is for water rinse with filtered water. Sink 3 is for disinfectant. Sink 4 is for rinse with filtered water. Each sink has a wall mounted tap above it.

This system is often found in France, where glutaraldehyde is currently used. In addition to the health and safety benefits of ClO₂ there is a desire to discontinue use of glutaraldehyde because it does not effectively destroy CJD prions and because of its toxicity to humans.

This variant would provide a dose of 400 - 2000 ppm into the disinfecting water to replace the glutaraldehyde. The unit may be mounted under the sink and ClO₂ may be piped up to the wall-mounted tap.

**Product variant 6 -** A stand alone irrigation unit supplying ClO₂ at a concentration of 20 ppm as a biocidal wash unit. Typical applications could be wound irrigation during surgery, hip replacement surgery, dentistry and for washing and disinfection of the feet of diabetics who are prone to foot infections. The mobile irrigation units would typically need to pump base and activator plus sterile water from 5 litre bottles.

Product variants 1 to 3 demand ClO₂ at a rate dictated by the washer disinfection machines, configurations 4 and 5 demand ClO₂ at a rate dictated by the mains water supply flow rate, and configuration 6 demands ClO₂ at a relatively slow flow rate dictated by the pump speed appropriate for those applications using a bottled water supply.

In order that the ClO₂ generator can service a variety of application needs, and have the minimum amount of stored, batch produced ClO₂, the rate at which we mix Base and Activator and allow it to react for 30 seconds will dictate the rate at which we can continuously supply ClO₂ into the water supply.

The highest flow rates for pumping mixed Base and Activator are encountered on applications requiring ClO₂ to be injected into the mains water supply at concentrations of 200 ppm. This concentration requires a dose rate of 13.3 ml of ClO₂ reagent mixture per litre of water. To prevent damage to flow meters and internal components within the ClO₂ generator system it is preferred to restrict flow to 15 - 20 litres per minute. The dosing rate will therefore be in the range 202 - 270 ml/min for these applications.

For endoscope washer disinfection machines the anticipated maximum rate at which ClO₂ must be manufactured by the Generator has been calculated at 40 ml of ClO₂ solution per minute. This dosing rate assumes the washers require 40 ml of ClO₂ reaction mixture to be injected into 30 litres of water in one minute for the rinse cycle and 160 ml of reaction mixture to be injected into 30 litres of water in four minutes for the decontamination cycle. These concentrations require a dose rate of 1.3 ml and 5.3 ml of ClO₂ reaction mixture per litre of water respectively.

This decontamination dosing rate assumes a ClO₂ concentration of 80 ppm rather than 200 ppm, based on a combined rinse dosing system and decontamination dosing system that provides the washer disinfector machines with a minimum dose of 20 ppm for all water entering the machine. It is likely that decontamination can be carried out with a lower ClO₂ dose of 80 ppm given that bacterial growth within the washer system will already have been minimised by the low dose 20 ppm rinse water.

### Summary:

A low dose (about 20 ppm) is needed for product variants 1, 2, 4 and 6. The dose concentration is about 1.3 ml/litre of H₂O at a flow rate 40 ml/minute. Dosing for the decontamination phase on a combined rinse/decontamination product (variant 2) will use a dose concentration of 5.3 ml of ClO₂ reaction mixture per litre of H₂O at a flow rate of 40 ml per minute.

A high dose (about 200 ppm) is appropriate for product variants 3 and 5. The dose concentration is about 13.3 ml/litre of H₂O, at a flow rate 40 ml/minute for product variant 3 and a maximum of 270 ml/minute for product variant 5.

Applications such as use in dental lines and wound irrigation, or to prevent build-up of biofilms in systems, may require ClO₂ concentrations lower than 20 ppm, typically about 5 ppm.

Other aspects and benefits of the invention will appear in the following specification, drawings and claims.

### BRIEF DESCRIPTION OF DRAWINGS

The invention will now be further described, by way of example, with reference to the following schematic drawings in which:
Figure 1 is a flow sheet for a method of producing a stream of aqueous ClO₂ sterilizing fluid in accordance with an embodiment of the present invention;
Figures 2 and 3 are flow sheets for methods of measuring ClO₂ concentration in a sterilizing fluid produced in accordance with embodiments of the present invention;
Figure 4 is a flow sheet for a method of producing a stream of aqueous ClO₂ sterilizing fluid in accordance with another embodiment of the present invention;
Figure 5 is a flow sheet for a method in accordance with a particularly preferred embodiment of the invention;
Figure 6 is a perspective view of an apparatus for producing aqueous chlorine dioxide in accordance with the method shown in Figure 5;
Figure 7 is a perspective view of the apparatus of Figure 6 with the cover off;
Figure 8 is a perspective view of the apparatus of Figure 7 with the control panel and electronics panel open;
Figure 9 is a perspective view of the apparatus of Figure 8 with the lower partition removed;
Figures 10 and 11 show the lower manifold assembly of Figure 8 as, respectively, an exploded view and a perspective view;
Figures 12 and 13 show the upper manifold assembly of Figure 8 as, respectively, an exploded view and a perspective view;
Figure 14 is an exploded view of the manifolds and bracket assembly of Figure 8;
Figure 15 is a perspective view of a sensor and holding tank arrangement for use with the apparatus of Figure 6;
Figure 16 illustrates the electrical architecture of a preferred embodiment of the invention.

### DETAILED DESCRIPTION

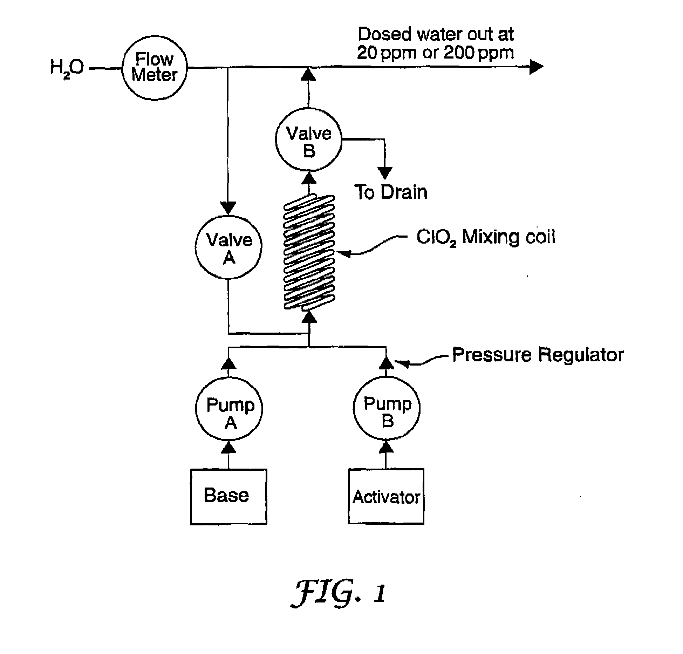

Referring to Figure 1, apparatus is shown for producing a stream of aqueous chlorine dioxide (ClO₂) sterilizing fluid for use in sterilizing medical and dental instruments or for wound irrigation or skin asepsis. The concentration of ClO₂ will be varied according the intended application. The apparatus comprises a first pump (Pump A) connected to a source of base and a second pump (Pump B) connected to a source of activator. In this example, the base and activator are comprised as set forth in Example 3 of EP 0 785 719. When the base and activator are mixed they react to produce aqueous ClO₂. The reaction mixture preferably has a pH in the range 4.5 to 6.5, notably 5.5 to 6.5.

The output from each pump connects to the proximal end of a tubular helical coil (ClO₂ mixing coil) which functions as an elongate reaction chamber. As pumping continues, reaction mixture is pumped progressively through the mixing coil to the distal end of the coil. With pumping continuing, the reaction mixture passes from through the mixing coil in about 30 seconds, during which time the reaction to form ClO₂ is substantially complete. With further pumping, the reaction mixture is injected into a conduit through which passes a stream of water. The reaction mixture mixes with the water to form a stream of aqueous ClO₂ sterilizing fluid. Depending on the rate of pumping, the concentration of ClO₂ in the sterilizing fluid can, in this example, be varied between about 20 ppm and about 200 ppm. Concentrations above or below this range may be generated either by varying the pumping rate, the water flow rate, or the concentration of reagents.

Each pump is a diaphragm pump, which uses an elastomeric diaphragm to draw fluid in and to expel fluid from the pumping chamber. On the down-stroke the diaphragm draws fluid into the chamber through an inlet valve and on the upstroke the diaphragm forces fluid out through an outlet valve. The pumps are self-priming. They can provide pumping pressures up to 600 kPa and flow rates in the range 0 to 300 ml/min. Advanced motor control technology speeds up the motor on the intake stroke to minimize fluid output pulsing. In this example, the pump body and valve components are made from stainless steel, and the diaphragms are coated with PTFE. A variety of motor and encoder options are available to drive and control operation of the pumps. In the present example, the pumps are operated under stepper motor control and monitored by means of optical encoders mounted on the motor shafts. The optical encoders measure graticules as they pass the optical sensor, giving precise measurement of shaft rotation, and hence volume pumped. The displacement volume for each pump per cycle is about 0.5 ml.

Each pump is provided with a pressure regulator (pressure control valve), which ensures that pumping is carried out against a substantially constant back pressure. The pressure control valve comprises a spring-biased piston against which the pump works. This arrangement ensures that the pump operates at a constant pressure regardless of the pressure in the water supply. To increase the working life of the pressure control valves it is preferred that the valve fittings are coated with PTFE or a fluoroelastomeric material which has good resistance to chemical attack. In the present example example, the back pressure is 4 bar (400 kPa), which is the same as the nominal mains water pressure in the conduit. All pumps are affected by back-pressure, which reduces the flow rate. By setting a constant back pressure, the pressure experienced by the pumps is kept constant, which in turn keeps the volume expelled during each pump cycle substantially constant. Thus, the apparatus enables precisely controlled quantities of reagents to be mixed and injected into the water stream, thereby providing a stream of sterilizing fluid with the concentration of ClO₂ controlled to within the limits demanded by particular applications within a medical or surgical facility. In a preferred embodiment, the spring pressure may be adjusted if desired, to permit corresponding adjustment to the bolus volume delivered by the pump.

PTFE tube connections are used to connect the pump outputs to the mixing coil, with stainless steel fittings used to provide greater resistance to damage from ClO₂. The valves are stainless steel solenoid valves with Viton® fluoroelastomer fittings. Suitable valves are sold by Parker Hannifin and Cole Palmer.

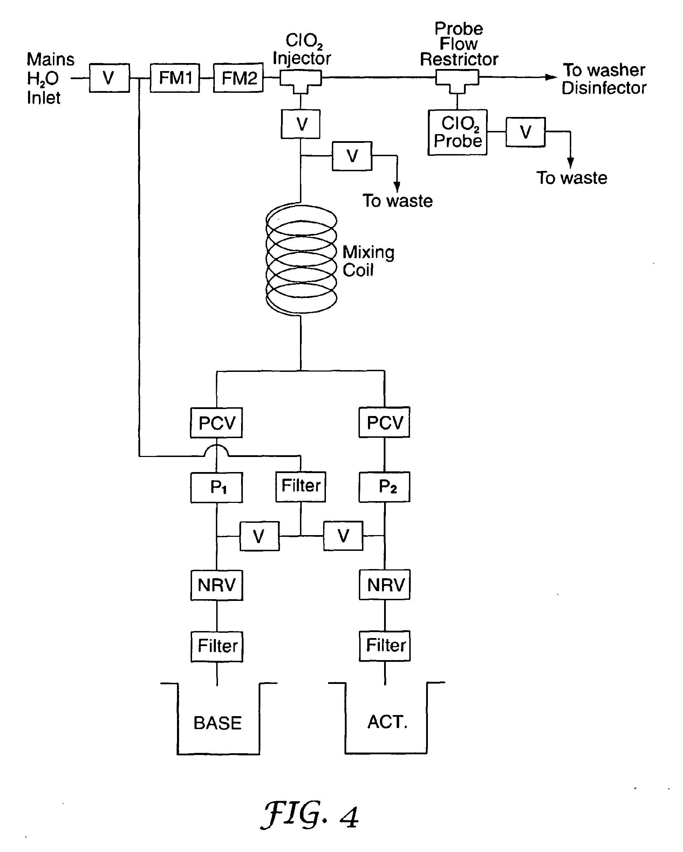

The mixing coil is a vertically-mounted stainless steel helical tube. The volume capacity of the coil is selected according to the intended application. For most applications, the mixing coil will have a 20 ml capacity. However, for Product Variant 5 the coil may have a 100 ml capacity, based on a preferred mixing time of 30 seconds. The distal end of the coil is connected to the water supply conduit via a T connector. A three-way valve (Valve B) isolates the top of the coil from the T connector. A two-way valve (Valve A) allows water to be introduced into the base (proximal end) of the coil. Operation of valves A and B allows the coil to be vented to drain and back-flushed with water. A flow meter in the conduit allows the necessary injection rate of ClO₂ into the water stream to be dynamically calculated and adjusted under software control (not shown). In a preferred embodiment, a second flow meter (FM2, as shown in Figure 4) is provided in series with the first flow meter to provide a confirmation that the correct water flow is being measured. If the measured flow from both meters differs by more than a predetermined amount, an alarm condition is triggered.

### Example 1:

The washer needs 40 ml of ClO₂ solution from the mixing coil to dose the rinse water of the 15 litre sink at the rate of 40 ml/minute. Base and Activator are injected into the base of the mixing coil by the two pumps. The internal volume of the coil is 20 ml. It takes 30 seconds for the Base and Activator to fill the coil. During the 30 seconds the Base and Activator are thoroughly mixed and ClO₂ is produced. The Base and Activator pump continue to push the 20 ml packet of ClO₂ out of the mixing coil and into the water line at 400 kPa at the demand rate of 40 ml/minute. At the end of the delivery cycle the mixing coil is still full of ClO₂. Software control may be used to determine whether to hold this ClO₂ in the coil, because there is an expectation that another batch will be required in a pre-determined period, or whether to flush it out using Base or water.

### Example 2:

The washer needs 160 ml of ClO₂ solution to dose both chambers of a washer disinfection machine at the rate of 40 ml/minute. We proceed exactly as Example 1 above but continue to mix and pump ClO₂ at the rate of 40 ml/minute until 160 ml of ClO₂ has been dispensed into the water supply. At the end of the delivery cycle the mixing coil can be flushed with Base or water or the ClO₂ retained as required.

### Verification of ClO₂ concentration using probe technology

Probes exist to measure ClO₂ concentrations in the range 1 - 20 ppm. Such a probe may be located in the sterilizing fluid stream and used to check that the correct concentration of ClO₂ is being generated for an application such as wound irrigation. However probes for measurement of ClO₂ in higher concentrations, for example in the range 100 - 200 ppm, tend to have limited lifetimes. As an alternative to using such higher concentration probes, a 1 - 20 ppm probe may be used to verify ClO₂ concentrations in all applications. This would require concentrations nominally higher than 20 ppm to be diluted before measurement, which creates a 'virtual' result rather than an absolute result measured directly from the sterilizing fluid output from the generator.

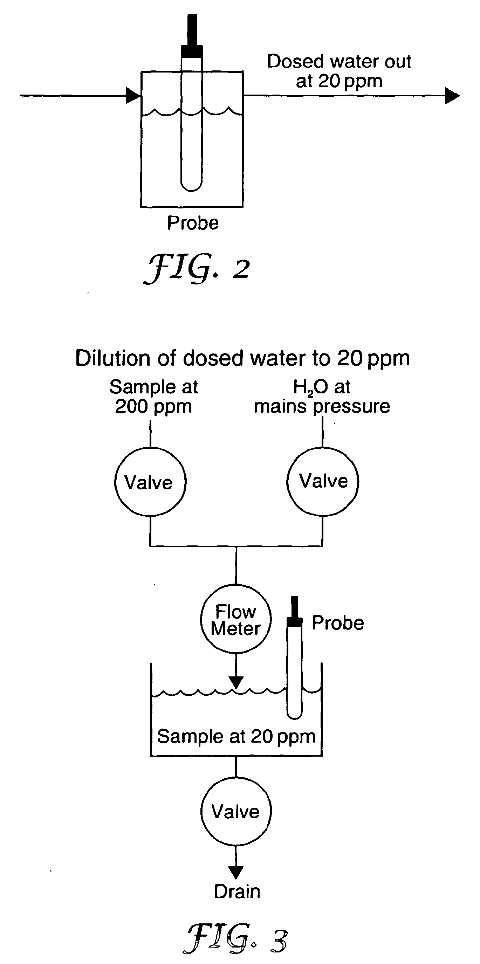

For measurement of ClO₂ at concentrations up to 20 ppm we can insert the probe directly into the dosed water stream, as illustrated in Figure 2.

For measurement of ClO₂ at concentrations greater than 20 ppm the dosed water would have to be accurately pre-diluted and an algorithm used to interpret the result. A suitable arrangement is illustrated in Figure 3. Here, sterilizing fluid is drawn into a dilution chamber and mixed at the correct ratio with plain water to bring the ClO₂ concentration to within the measuring range of the probe. In a gravity fed system, valves and a flow meter are used to control the dilution. Alternatively, a pump may be used to accurately dispense sterilizing fluid and plain water into the chamber in the correct quantities.

By changing the ClO₂ concentration, or flushing the system with water, from time to time, a check could be made that the probe has not become "struck" at the required concentration.

Referring now to Figure 4, an alternative arrangement is shown, in this example for providing ClO₂ sterilizing fluid to a washer disinfector. Base and activator are fed to their respective pumps (P1 and P2) via a non-return valve and a filter. To facilitate priming of the pumps and optionally purging of the system, for example to remove crystals or sediment, a line is taken from the mains water supply conduit to provide filtered mains water to the pumps via valves V.

Thus, the entire system can be flushed with mains water, including the pumps.

The distal end of the ClO₂ mixing coil is connected to a ClO₂ injector via a valve V. Reagent mixture is injected into the mains water stream by the injector. Because diaphragm pumps do not pump continuously, there will tend to be a cyclic variation in the quantity of ClO₂ which is injected into the water supply when the pumps are in phase. The problem may be reduced or eliminated by operating the pumps alternately. In the present example, to avoid an in-line ClO₂ probe momentarily measuring a concentration which is not characteristic of the actual concentration in the washer disinfector, and triggering an alarm condition, the probe is located in a small reservoir connected to the mains conduit by a T-connector flow restrictor. The restrictor passes most of the sterilizing fluid to the washer disinfector, but continually diverts a small quantity (for example 3-5%) to the reservoir where the probe is located. The concentration of ClO₂ in the reservoir will be representative of that in the washer disinfector. To permit time for a sufficient quantity to be collected in the reservoir the probe may be located near the top of the reservoir so that it only measures ClO₂ concentration in fluid that has initially mixed in the reservoir. Fluid which has passed the probe may be vented to waste (as illustrated in Figure 4) or to the washer disinfector.

Alternatively, the probe reservoir could be fully filled in the same time that the washer sink is filled, and the probe then activated prior to carrying out washing/disinfecting. For example, a 15 litre washer sink and a 0.5 litre probe reservoir may fill simultaneously, the ratio being 30:1.

Only when both have filled would the ClO₂ concentration be measured.

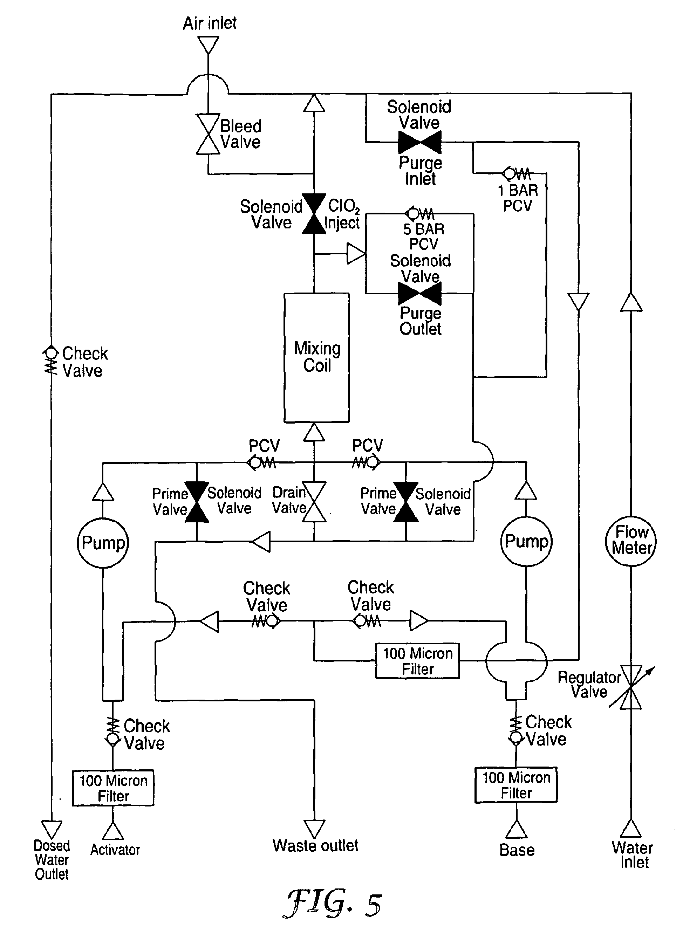

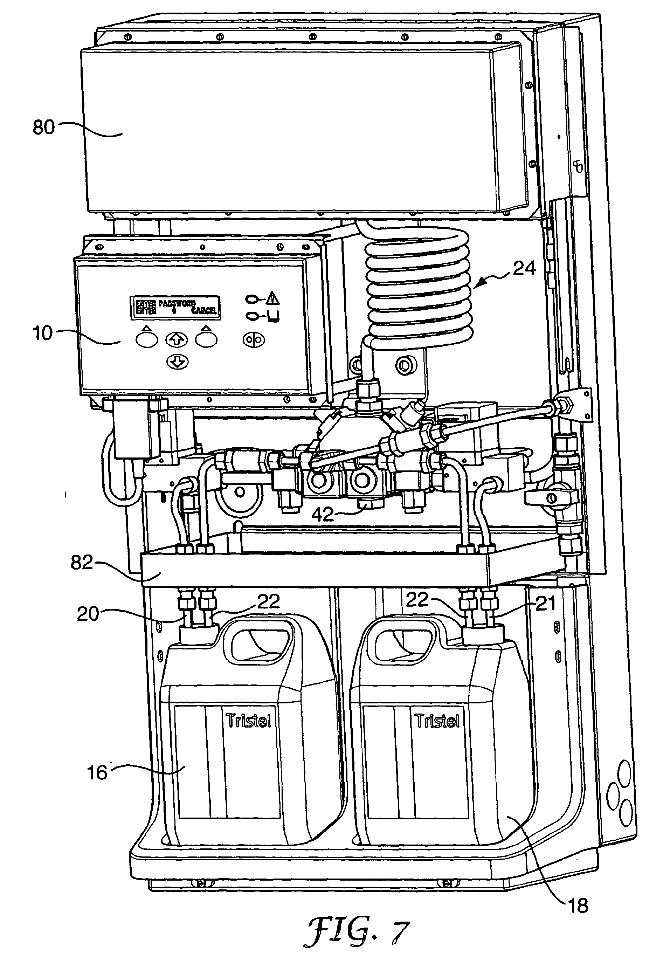

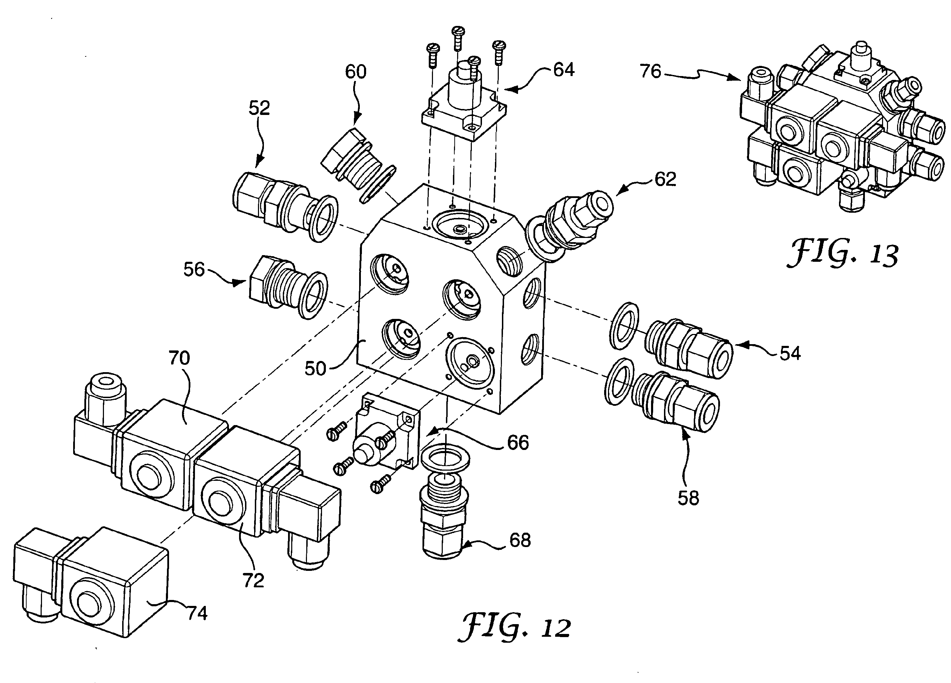

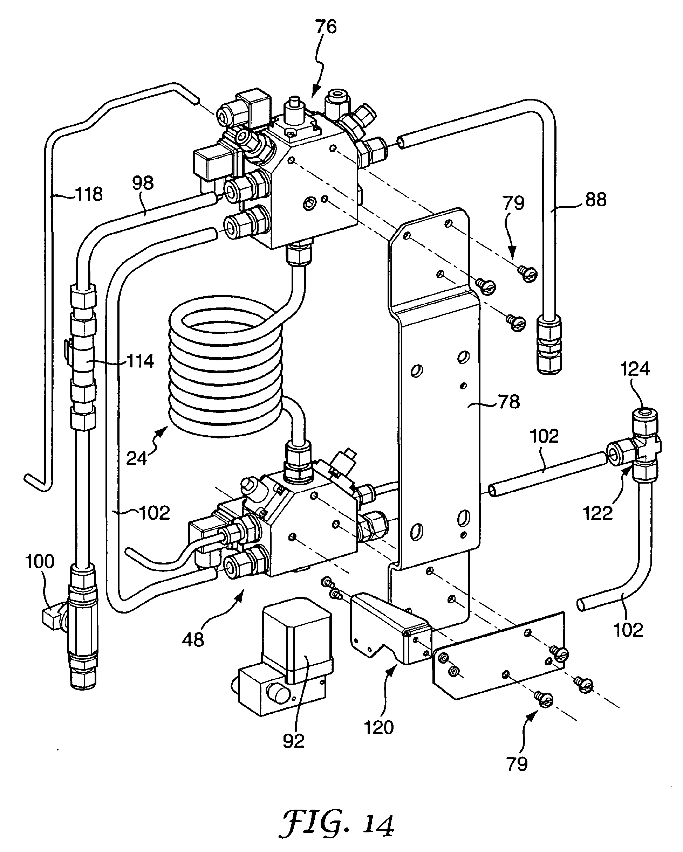

A particularly preferred embodiment of the invention will now be described with reference to Figures 5 to 16. In the flow sheet of Figure 5 the tubing which carries the activator and base solutions to their respective pumps is provided with a 100 µm filter and a check valve to ensure one-way travel of fluid. The base and activator are pumped against a back pressure of 350 kPa to the mixing coil where they mix and travel through the mixing coil as previously described. The reaction mixture takes about 30 seconds to travel to the top of the coil, by which time the reaction to form ClO₂ is substantially complete. The ClO₂ solution is injected into the mains water supply *via* a solenoid valve. If air is initially in the system this may be bled off via a bleed valve.

In this embodiment, the dosed water outlet line has a check valve, which ensures that water will not pass through until a preset pressure of 100 kPa is reached. This arrangement ensures that the system is always pressurized when in use.

The system includes an arrangement for priming and purging the system. Priming the system involves initially filling the diaphragm pumps with water so that they can operate on liquids, which they pump efficiently, and not air, which can cause diaphragm pumps problems. Purging the system involves flushing aqueous ClO₂ out with water to prevent long-term contact of the internal surfaces with ClO₂, which is desirable to increase the operating lifetime of the system. To initiate purging, the solenoid purge valve is opened, permitting water to pass through a purge line to the tubing which connects the base and activator sources to the diaphragm pumps. The purge water passes through a 100 µm filter, and check valves ensure that the purge water passes up through the pumps and into the mixing coil. With the ClO₂ injection solenoid valve closed, and a purge outlet solenoid valve open, purge water passes from the coil and leaves through a waste outlet. To prime the pumps without purging the coil, the solenoid prime valves are opened so that water which passes through the pumps goes directly to the waste outlet. A 100 kPa check valve connects the output from the purge inlet to waste, thereby limiting purge water pressure. Similarly, a 500 kPa check valve is provided to vent reaction products from the mixing coil to waste to prevent dangerous excess of pressure if the ClO₂ injection valve and the purge outlet valves are closed. A drain valve is provided to permit manual draining of the coil to waste.

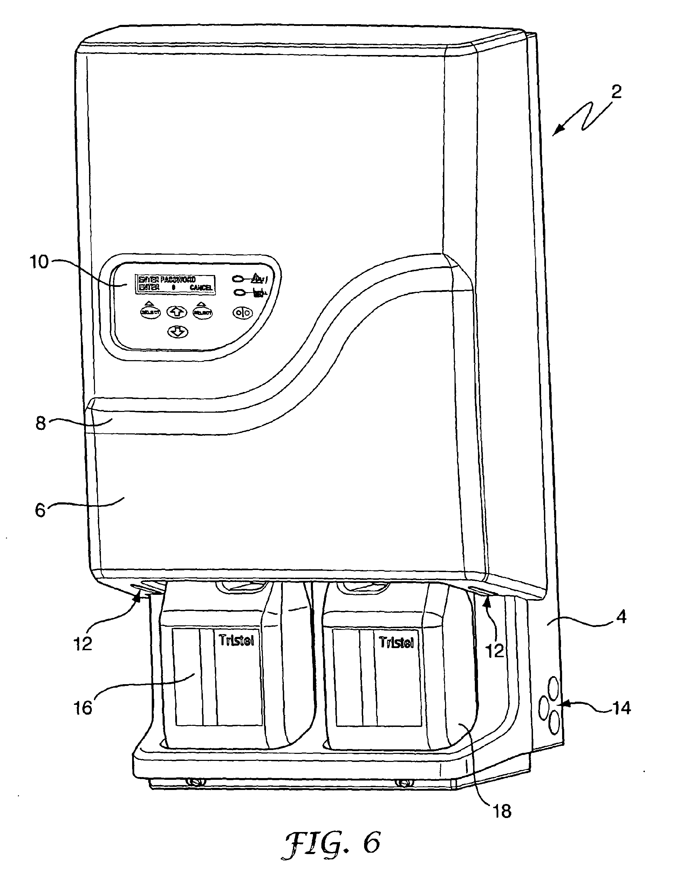

Referring now to Figures 6-15, a particularly preferred embodiment of apparatus 2 for generating ClO₂ sterilizing fluid is described by way of non-limiting example only. The apparatus 2 has a chassis 4 of C-section folded stainless steel and a cover 6. In this embodiment a user-accessible control panel 10 is located above a decorative moulding 8. The cover 6 is releasably secured to the chassis 4 by means of screws (not shown) in slots 12 of the cover 6. Holes 14 are provided on both sides of the chassis 4 for pipe connections for water inlet, sterilizing fluid outlet, and waste outlet. Only the holes on one side will typically be used, but holes are provided on both sides for flexibility of installation. Alternatively, pipe connections may be taken out through the bottom of the chassis. Grommets are provided in the holes 14 that are not used for the pipes. The apparatus may be wall mounted by means of fixings 126 in the chassis 4.

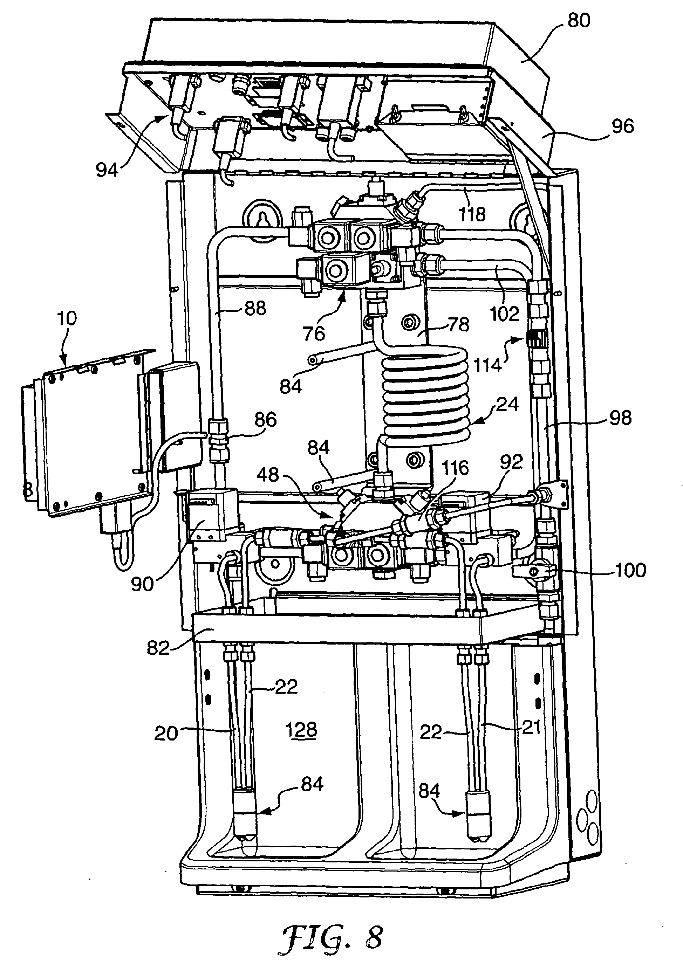

The control panel 10 is releasably secured to a pair of standoffs 84 by releasable securing means, in this example, by bolts (not shown). With the cover 6 off, the bolts can be removed and the control panel can be swung outwardly as shown in Figure 8. A cover 80 encloses electronics components and assemblies 94 for operating the apparatus, including solenoid valves, pumps, a flow meter, and bottle level sensors. A lead acid battery 96 is provided as a backup in case of mains power failure. If mains power fails, the battery 96 will power a mains failure alarm and ensure orderly shutdown of the system.

The activator and base solutions are provided in bottles 16, 18. Fluid from the bottles 16, 18 travels through Tygon® flexible tubing 20, 21 to pumps 90, 92. The tubing 20, 21 terminates in a bobbin 84 which is open at the bottom to allow fluid entry from the bottles 16, 18. Each bobbin 84 contains (Figure 5) a 100 µm stainless steel filter at the bottom and a non-return valve at the top. Purge water tubing 22 is connected to the top of each bobbin 84, above the non-return valve, to permit purge or priming water to pass through the tubing 20, 21 and up through the pumps 90, 92.

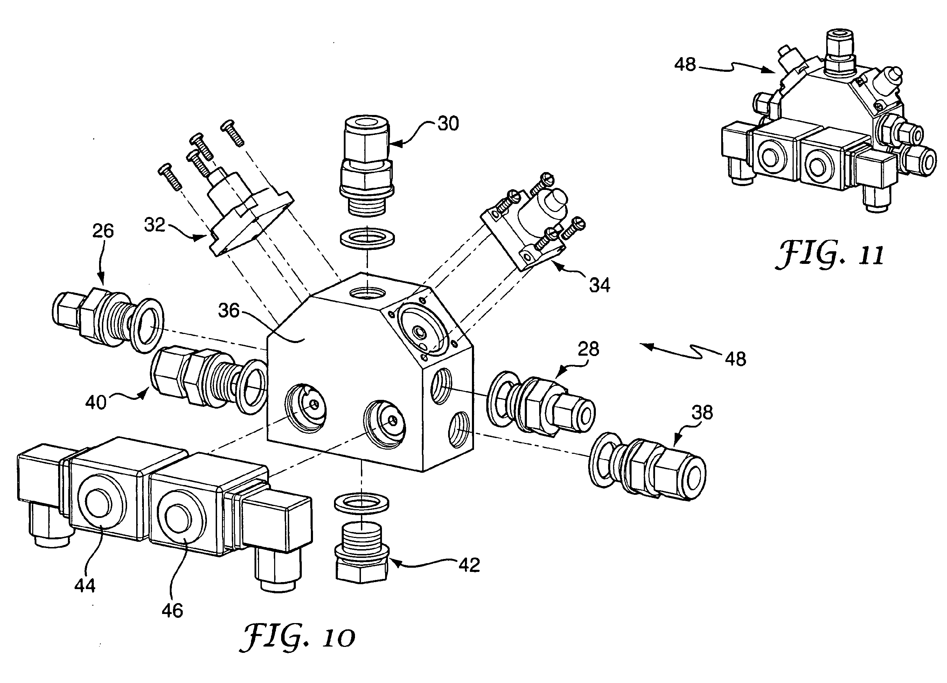

The activator pump 90 and base pump 92 are connected to a lower manifold assembly 48 via an activator pump inlet 26 and a base pump inlet 28, as best shown in Figure 10. The base and activator solutions mix in the lower manifold housing 36 and pass to the mixing coil 24 through a lower coil fitting 30. An 'activator' pressure control valve 32 and a 'base' pressure control valve 34 provide a constant back-pressure for the pumps 90, 92, as previously described. In the preferred embodiment, the streams of base and activator meet 'head-on' which we have found to give efficient mixing, before being diverted up a single passageway normal to the direction of the streams. A drain valve 42 is provided at the bottom of the lower manifold housing 36 for manually draining the contents of the manifold housing 36 and coil 24. Fluid from the drain valve 42 is received in a tray 82 and passes to a waste outlet 108 through a drain hole (not shown) in the tray 82 and a T-piece 112 connected to the drain hole. Also provided in the lower manifold assembly 48 are an activator prime valve 44 and a base prime valve 46. The prime valves 44, 46 are opened during priming of the pumps 90, 92. The lower manifold housing 36 is also provided with a waste inlet 38 for receiving waste fluids from an upper manifold assembly 76, which will be described below, and a lower manifold waste outlet 40. Waste fluid passes from the lower manifold waste outlet 40 to the waste outlet 108 *via* waste outlet pipe 102 (Figure 14).

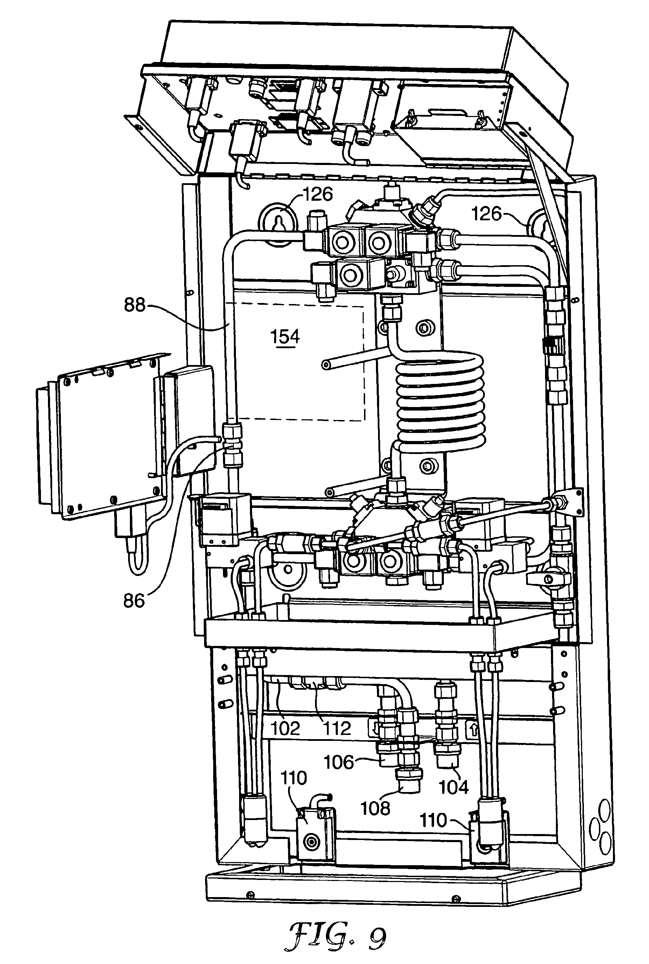

The top of the mixing coil 24 is connected to an upper manifold assembly 76 through a reaction mixture inlet 68 on the upper manifold housing 50. This housing 50 receives mains water from a mains inlet pipe 98 via a mains inlet 54. Opening of a ClO₂ injection valve 70 permits injection of the reaction mixture into the mains water stream to form a sterilizing fluid which exits the upper manifold housing 50 *via* a sterilizing fluid outlet 52. The sterilizing fluid passes through an output pipe 88 to sterilizing fluid outlet 106 (Figure 9). An optional check valve 86 is provided in the output pipe 88 to ensure the system is always pressurized when in use. A blanking plug 60 blocks an outlet which communicates with the mains water supply. This plug 60 may be replaced by a conduit to supply water to a sensor assembly for monitoring ClO₂ concentration in the fluid stream, as will be described later.

A purge water inlet 62 is connected to the mains inlet pipe 98, for channelling water for priming or purging of the system through a purge line 118 when a purge inlet solenoid valve 72 is opened. Purge water from the inlet 62 is fed through a 100 µm filter 116 to the purge water tubing 22 for priming of the pumps 90, 92 and optional purging of the system as previously described. A purge inlet pressure relief valve 64 is provided to limit purge water pressure, in this example to 100 kPa, by diverting purge water to the waste pipe 102.

An air inlet 56 is provided to permit ingress of air or venting of air from the system, via a bleed valve (not shown).

An upper waste outlet 58 communicates with reaction mixture inlet 68 from the top of the mixing coil via a purge outlet solenoid valve 74. This valve 74 is opened when the system is being purged, and feeds purge water from the mixing coil to the lower manifold waste inlet 38 via the waste pipe 102. A purge outlet pressure relief valve 66, in this example having a 500 kPa burst pressure, is provided in parallel. If pressure in the coil 24 exceeds the specified value, the pressure relief valve 66 opens to permit pressurised fluid to escape to waste.

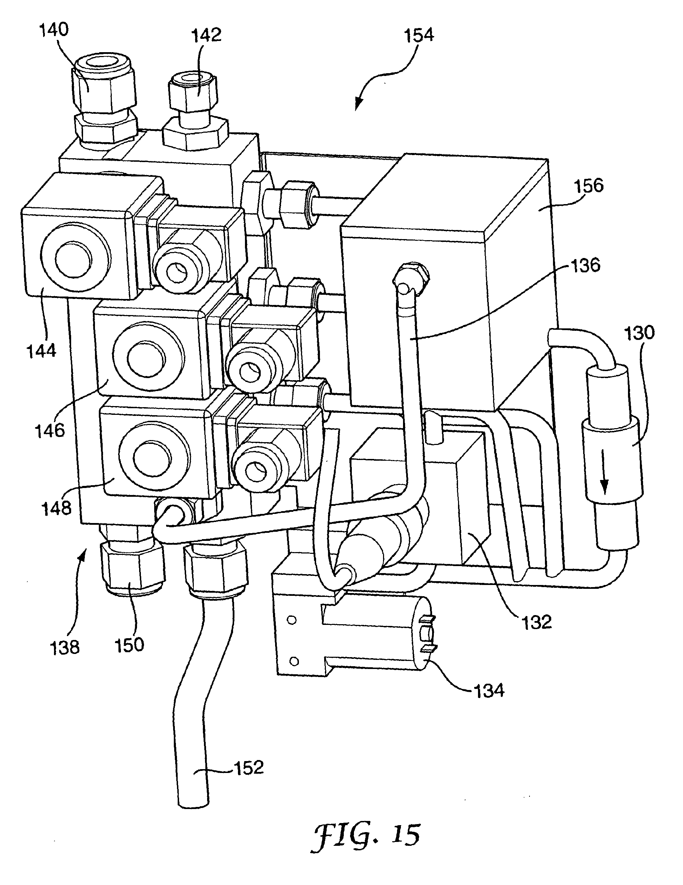

Referring now to Figure 14, the upper manifold assembly 76 and the lower manifold assembly 48 are each secured to a mounting bracket 78 by bolts 79. The base pump 92 is secured to the mounting bracket 78 by a base pump bracket assembly 120. Mains water flow through the mains inlet pipe 98 is controlled by a tap 100 and measured by a flow meter 114. The waste outlet pipe 102 includes a T-piece 122 which is closed at the top by a blanking plug 124. This plug 124 may be removed and the top of the T-piece 124 may be connected to the waste outlet of an optional sensor assembly 154, a preferred example of which is described below with reference to Figure 15 and which may be located in the chassis where indicated by the broken lines (Figure 9).

The purpose of the sensor assembly 154 is to maintain a ClO₂ sensor immersed in water until a ClO₂ concentration reading is to be taken, and then to flush out the ClO₂ solution and leave the sensor in clean water. This is desirable because commercial ClO₂ sensors tend to become saturated, or 'blinded' after more than about two minutes of immersion in aqueous ClO₂. The preferred arrangement ensures that regular ClO₂ concentration measurements can be taken (each measurement taking less than two minutes) without the sensor 132 becoming saturated. The assembly 154 has a waste outlet pipe 152 for connection to the top of the T-piece 124 in the waste outlet pipe 102. The assembly 154 comprises a manifold block 138 connected to a 100 ml holding tank 156, a sensor unit 132 and a circulation pump 134. The sensor unit 132 comprises a housing in which the sensor (not shown) is located. The pump 134 keeps fluid circulating through the sensor unit 132. A check valve 130 prevents backflow from the sensor unit 132 into the holding tank 156. The holding tank 156 has an overflow pipe 136 which is connected to the waste pipe 152.

The manifold block 138 has a water inlet 142 for introducing purge water into the sensor unit 132 under control of a water inlet solenoid valve 146. The water inlet 142 receives purge water from the water outlet on the upper manifold housing 50 which is otherwise blocked off by the plug 60. A water outlet solenoid valve 148 controls fluid output to waste 152. The manifold block 138 has a sterilizing fluid inlet 140 and a sterilizing fluid outlet 150 for connection in the sterilizing fluid outlet pipe 88. Thus, sterilizing fluid normally flows through the manifold block 138. Operation of a sterilizing fluid input solenoid valve 144 diverts the passage of sterilizing fluid from the inlet 140 to the holding tank 156. When a ClO₂ concentration reading is to be taken the water inlet valve 146 is operated to close off the water inlet 142. The sterilizing fluid input valve 144 is opened long enough to allow sterilizing fluid to flow into the holding tank 156 for circulation through the sensor unit, and the water outlet valve 148 is opened to allow water to leave the system and be replaced by sterilizing fluid. The fluid capacity of the holding tank 156 is much greater than that of the sensor unit 132 so that water in the sensor unit 132 is quickly substantially replaced by sterilizing fluid. The ClO₂ concentration reading is taken and the water outlet valve 148 is opened to allow sterilizing fluid to be pumped out of the sensor unit 132. The water inlet valve 146 is then opened and water is pumped through the sensor unit 132 until the sterilizing fluid has been pumped to waste. After the holding tank 156 has emptied and the sensor unit 132 is again filled with undosed water, the outlet valve 148 is closed.

Referring now to Figure 9, a level sensor 110 is provided for each bottle 16, 18. In this example, the level sensor 110 is of a type known *per* se which works by capacitance measurement. Other level sensors, such as optical sensors, may be used. Behind a lower partition (Figure 8) are located the mains inlet 104, sterilizing fluid outlet 106, and waste outlet 108 for connection to pipework which will be disposed through selected holes in a side of the chassis 4, or through the bottom of the chassis.

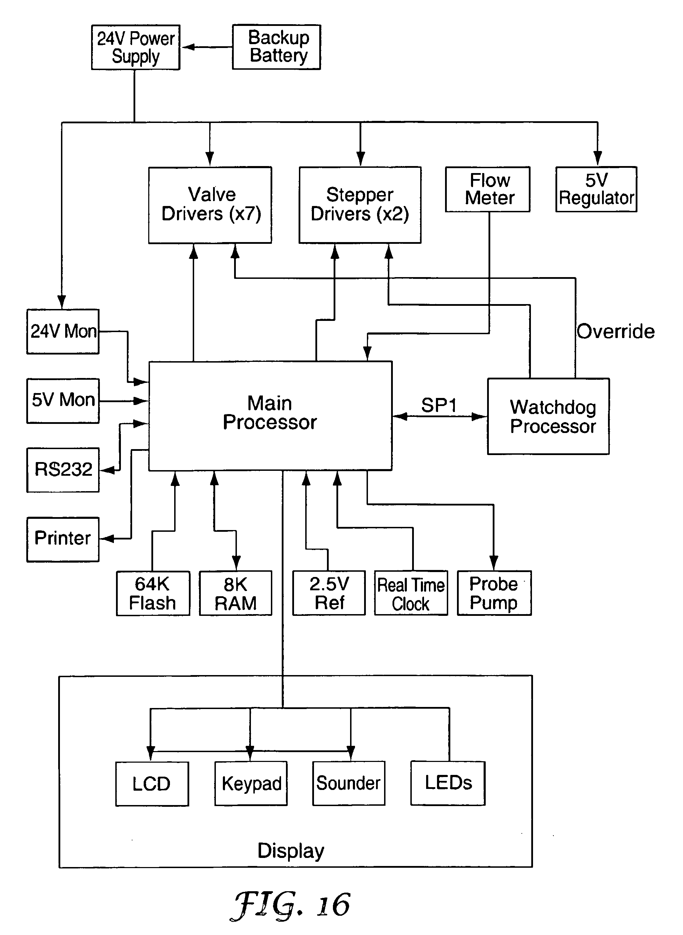

Suitable means for automating the operations of the apparatus will be well known to those skilled in the art of electronics. Details of a preferred electrical architecture for the apparatus are given by way of example in Figure 16. The control panel includes a keypad for data and command entries, an alphanumeric LCD for displaying user information data, an 'alarm' LED, a "bottle empty' LED and a 'dosing' LED, together with a sounder for producing an audible alarm in the case of system failure. These functions are controlled by a main processor, in this example a Motorola MC68HC11K1 8-bit microcontroller running in expanded mode, with 64K Flash memory and 8K RAM. The main processor controls the valve drivers, stepper drivers, and probe pump on the sensor unit. It has RS232 and printer interfaces, and a real time clock to allow for the programming of dosage regimes at specific times. A watchdog processor, for example a Motorola MC68HC11K1 8-bit microcontroller, running in expanded mode, is linked to the main processor by and SPI interface. The watchdog processor receives service messages from the main processor and uses these messages to verify correct operation of the main processor. If an error condition is detected, the watchdog puts the system in safe mode. The electronics are powered by 24 volts from the main switched-mode power supply. This provides power to the valve and motor circuitry and the bottle fluid level detectors.

The remainder of the system runs from 5 volts, which is generated by an on-board switched-mode step-down regulator. The main processor monitors the 24 volt power supply voltage to verify that it is within required limits and to detect when the system if running from (12 V) battery power. The main processor also monitors the 5 V supply by applying this to the ADC reference input and comparing with a 2.5 V reference voltage.

Thus, the preferred embodiment provides for accurate pumping of reagents to the reaction coil, which enables the production of ClO₂ solution at the correct concentration and an appropriate dwell time in the coil to enable substantially complete reaction. The pumping rates can be varied according to the measured water flow rate to ensure that the concentration of ClO₂ in the sterilizing fluid is within a desired range for the particular application, and this concentration may also be confirmed by the use of suitable probe technology. The system is therefore ideal for use in a medical facility, where accurate concentration control is needed. Moreover, the preferred reagent mixture avoids the use of strong acids and is physiologically well tolerated.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately, or in any suitable combination.

## Claims

1. A method of producing a stream of aqueous chlorine dioxide sterilizing fluid suitable for use in sterilizing medical and dental equipment or for wound irrigation or skin asepsis, the method comprising:
pumping fluid portions of a first reagent and a second reagent into the proximal end of an elongate reaction chamber to form a reaction mixture, the reagents being selected to react together to form aqueous chlorine dioxide;
providing a stream of water around or adjacent to the distal end of the reaction chamber;
continuing to pump fluid portions of said reagents so as to cause the reagent mixture to travel through the reaction chamber and then into the stream of water to form a stream of aqueous chlorine dioxide sterilizing fluid;
wherein the pumping rates and the internal dimensions of the elongate reaction chamber are selected so that the reaction to form chlorine dioxide is substantially complete when the reagent mixture exits the reaction chamber; and
continuing pumping fluid portions of said reagents until a desired quantity of said sterilizing fluid has been produced;
wherein the pumping of each reagent is carried out by means of a diaphragm pump or a piston pump via a pressure-control valve so that each fluid portion is pumped at substantially constant pressure.

2. A method according to claim 1, wherein the pumps are operated under stepper motor control.

3. A method according to claim 2, wherein the stepper motor shaft for each pump is provided with an optical encoder for use in a closed loop feedback control arrangement.

4. A method according to any preceding claim, further including the step of measuring the flow rate of the stream of water, calculating the amount of ClO₂ required to be added to the stream to produce a desired concentration of ClO₂ in the sterilizing fluid, and adjusting the pumping rates if necessary to achieve said desired concentration.

5. A method according to any of claims 1-3, further including the steps of measuring ClO₂ concentration in the sterilizing fluid, comparing the measured concentration with a desired concentration, and adjusting the pumping rates if necessary to achieve said desired concentration.

6. A method according to claim 4 or claim 5, further including the step of triggering a visible or audible signal to indicate whether the sterilizing fluid is within a desired concentration range.

7. A method according to claim 4, wherein the step of measuring the flow rate comprises measuring a first flow rate at an upstream location and a second flow rate at a downstream location in the stream of water, and the method further comprises triggering an alarm condition in the event that the first and second flow rates differ by more than a predetermined value.

8. A method according to any preceding claim, wherein the elongate reaction chamber is a helical coil.

9. A method according to any preceding claim, wherein the first reagent is an aqueous solution of a chlorite, and the second reagent is an aqueous acidic solution.

10. A method according to claim 9, wherein the first reagent comprises aqueous sodium chlorite and the second reagent comprises citric acid, sorbic acid and boric acid.

11. A method according to claim 10, wherein the second reagent further comprises a copper and brass corrosion inhibitor, a steel and aluminium corrosion inhibitor, and a buffering agent.

12. A method according to claim 11, wherein the copper and brass inhibitor is a triazole or benzotriazole present in a weight percent of 0.01 to 2.0.

13. A method according to claim 11 or claim 12, wherein the steel and aluminium corrosion inhibitor is selected from the group comprising phosphates, molybdates and nitrates, and is present in a weight percent of from 0.01 to 5.0.

14. A method according to any preceding claim, wherein at least one of the first and second reagents further comprises a wetting agent, a defoamer or a combination thereof.

15. A method according to any preceding claim, wherein when equal fluid portions of the first and second reagents are mixed the resulting reagent mixture will have a pH in the range 4.5 to 6.5.

16. A method according to any preceding claim, wherein the pumping rate and the internal dimensions of the reaction chamber are selected such that the time taken for the reagent mixture to travel from the proximal end to the distal end of the reaction chamber is in the range 20 to 40 seconds.

17. A method according to any preceding claim, wherein the pressure control valve produces a back pressure in the range 300 to 500 kPa.

18. A method according to any preceding claim, wherein fluid portions of said first reagent and said second reagent are pumped simultaneously to said reaction chamber.

19. A method according to any preceding claim, wherein said stream of water is directed through a pressure control valve to ensure that the water is at a desired minimum pressure

20. Apparatus for producing aqueous chlorine dioxide from reaction of a first reagent and a second reagent, said aqueous chlorine dioxide being capable of dilution to form a sterilizing fluid suitable for use in sterilizing medical or dental equipment or for wound irrigation or skin asepsis, the apparatus comprising:
a first pump for connecting to a fluid source of said first reagent;
a second pump for connecting to a fluid source of said second reagent;
an elongate reaction chamber having proximal and distal ends;
both pumps having outputs in fluid connection with said proximal end;
wherein operation of the pumps when connected to the fluid sources will feed portions of the first and second reagents initially to the proximal end of the elongate reaction chamber to form a reaction mixture and wherein continued operation of the pumps will progressively pump the reaction mixture through the reaction chamber to the distal end and then eject the reaction mixture from the distal end;
wherein each pump is a diaphragm pump or a piston pump and is provided with a pressure-control valve in the fluid path between the pump and the reaction chamber so that each fluid portion will be pumped at substantially constant pressure.

21. Apparatus according to claim 20, further comprising a conduit connected to a source of flowing water, the conduit being in fluid connection with the distal end of the reaction chamber for receiving reaction mixture therefrom to form a stream of aqueous chlorine dioxide sterilizing fluid suitable for use in sterilizing medical equipment or wound irrigation.

22. Apparatus according to claim 20 or claim 21, further comprising a first container which contains a fluid comprising said first reagent and a second container containing a fluid comprising said second reagent; wherein said first container is in fluid connection with an input of the first pump and wherein said second container is in fluid connection with an input of the second pump.

23. Apparatus according to any of claims 20-22, wherein each pump is provided with a vent valve for priming of the pump.

24. Apparatus according to any of claims 20-23 wherein each pressure control valve produces a back pressure in the range 300 to 500 kPa.

25. Apparatus according to any of claims 20-24, wherein the pumps are provided with stepper motors for controlling operation of the pumps.

26. Apparatus according to claim 25, wherein the stepper motor shaft for each pump is provided with an optical encoder for use in a closed loop feedback control arrangement.

27. Apparatus according to claim 21, wherein the conduit is provided with a first flow meter for measuring the flow rate of water from the source; the apparatus further comprising means for calculating the amount of ClO₂ required to be added to the stream to produce a desired concentration of ClO₂ in the sterilizing fluid at the measured flow rate, and means for adjusting the pumping rates if necessary to achieve said desired concentration.

28. Apparatus according to claim 21, wherein the conduit is provided downstream of the distal end of the reaction chamber with a probe for measuring ClO₂ concentration; the apparatus further comprising means for adjusting the pumping rates if necessary so that the measured ClO₂ concentration falls within a desired range.

29. Apparatus according to claim 27 or claim 28, further comprising means for triggering a visible or audible signal to indicate whether the sterilizing fluid is within a desired concentration range.

30. Apparatus according to claim 27, wherein the conduit is provided with a second flow meter in series with the first flow meter; and wherein the apparatus further comprises means for comparing output from both flow meters to produce a compared value and for triggering an alarm condition in the event that the compared value exceeds a predetermined value.

31. Apparatus according to any of claims 20-30, wherein the elongate reaction chamber is a helical coil.

32. Apparatus according to any of claims 20-31, further comprising valve-controlled means for introducing a purging fluid into the reaction chamber and for draining fluid from the reaction chamber.

33. Apparatus according to claim 21, further comprising a valve-controlled fluid connection between the conduit upstream of the distal end of the reaction chamber, and the proximal end of the reaction chamber for feeding purging water into the reaction chamber.

34. Apparatus according to claim 33, further comprising a valve-controlled drain connection from the reaction chamber at or adjacent to the distal end, for feeding fluid from the reaction chamber to a drain.

35. Apparatus according to claim 28, wherein the probe is located in a reservoir arranged and adapted to continuously collect a sample part of the sterilizing fluid which passes through the conduit.

36. Apparatus according to claim 20, further comprising a sensor for measuring chlorine dioxide concentration in a sensor housing, means for selectively directing water into the sensor housing to keep the sensor wet, means for draining liquids from the sensor housing, and means for selectively directing said aqueous chlorine dioxide into said sensor housing to allow the concentration of chlorine dioxide to be measured.

## Patentansprüche

1. Verfahren zur Erzeugung eines Stroms von wässriger Chlordioxid-Sterilisationsflüssigkeit zur Verwendung bei der Sterilisierung von medizinischen und zahnärztlichen Geräten oder zur Wundspülung oder aseptischen Hautbehandlung, wobei das Verfahren Folgendes umfasst:
Pumpen von Flüssigkeitsanteilen eines ersten Reagenz und eines zweiten Reagenz in das proximale Ende einer länglichen Reaktionskammer zur Bildung eines Reaktionsgemisches, wobei die Reagenzien so ausgewählt werden, dass sie zur Bildung von wässrigem Chlordioxid miteinander reagieren;
Bereitstellen eines Wasserstroms um das oder neben dem distalen Ende der Reaktionskammer;
Fortgesetztes Pumpen von Flüssigkeitsanteilen der Reagenzien, damit das Reagenzgemisch durch die Reaktionskammer und dann in den Wasserstrom läuft, um einen Strom aus wässriger Chlordioxid-Sterilisationsflüssigkeit zu bilden;
wobei die Pumpgeschwindigkeiten und die inneren Abmessungen der länglichen Reaktionskammer so gewählt sind, dass die Reaktion zur Bildung von Chlordioxid im Wesentlichen abgeschlossen ist, wenn das Reagenzgemisch aus der Reaktionskammer austritt; und
wobei das Pumpen der Flüssigkeitsanteile der Reagenzien fortgesetzt wird, bis eine gewünschte Menge der Sterilisationsflüssigkeit erzeugt ist;
wobei das Pumpen jedes Reagenz mithilfe einer Membranpumpe oder einer Kolbenpumpe über ein Druckregelventil durchgeführt wird, so dass jeder Flüssigkeitsanteil im Wesentlichen mit konstantem Druck gepumpt wird.

2. Verfahren nach Anspruch 1, wobei die Bedienung der Pumpen von einem Schrittmotor gesteuert wird.

3. Verfahren nach Anspruch 2, wobei der Schrittmotorschaft für jede Pumpe mit einem optischen Codierer zur Verwendung in einer geschlossenen Feedback-Regelungsanordnung ausgerüstet ist.

4. Verfahren nach einem der vorhergehenden Ansprüche, ferner umfassend den Schritt der Messung der Strömungsgeschwindigkeit des Wasserstroms, der Berechnung der erforderlichen ClO₂-Menge, die dem Strom zugefügt werden muss, um eine erwünschte ClO₂-Konzentration in der Sterilisationsflüssigkeit zu erhalten, und der Einstellung der Pumpengeschwindigkeiten, sofern notwendig, um die gewünschte Konzentration zu erhalten.

5. Verfahren nach einem der Ansprüche 1-3, ferner umfassend die Schritte der Messung der ClO₂-Konzentration in der Sterilisationsflüssigkeit, des Vergleichens der gemessenen Konzentration mit einer gewünschten Konzentration und des Einstellens der Pumpengeschwindigkeiten, sofern notwendig, um die gewünschte Konzentration zu erhalten.

6. Verfahren nach Anspruch 4 oder Anspruch 5, ferner umfassend den Schritt des Auslösens eines sichtbaren oder hörbaren Signals, um anzuzeigen, ob die Sterilisationsflüssigkeit innerhalb eines gewünschten Konzentrationsbereichs liegt.

7. Verfahren nach Anspruch 4, wobei der Schritt der Messung der Strömungsgeschwindigkeit die Messung einer ersten Strömungsgeschwindigkeit an einer stromaufwärts liegenden Stelle und einer zweiten Strömungsgeschwindigkeit an einer stromabwärts liegenden Stelle im Wasserstrom umfasst, und wobei das Verfahren weiterhin das Auslösen eines Alarmzustands umfasst, wenn die ersten und zweiten Strömungsgeschwindigkeiten um mehr als einen vorbestimmten Wert abweichen.

8. Verfahren nach einem der vorhergehenden Ansprüche, wobei die längliche Reaktionskammer eine spiralförmige Spule ist.

9. Verfahren nach einem der vorhergehenden Ansprüche, wobei das erste Reagenz eine wässrige Lösung eines Chlorits ist, und worin das zweite Reagenz eine wässrige saure Lösung ist.

10. Verfahren nach Anspruch 9, wobei das erste Reagenz wässriges Natriumchlorit und das zweite Reagenz Zitronensäure, Sorbinsäure und Borsäure umfasst.

11. Verfahren nach Anspruch 10, wobei das zweite Reagenz ferner einen Kupfer- und Messing-Korrosionshemmer, einen Stahl- und Aluminium-Korrosionshemmer und ein Puffermittel umfasst.

12. Verfahren nach Anspruch 11, wobei der Kupfer- und Messing-Korrosionshemmer ein Triazol oder ein Benzotriazol in einem prozentualen Gewichtsverhältnis von 0,01 bis 2,0 ist.

13. Verfahren nach Anspruch 11 oder Anspruch 12, wobei der Stahl- und Aluminium-Korrosionshemmer aus der aus Phosphaten, Molybdaten und Nitraten bestehenden Gruppe ausgewählt ist und in einem prozentualen Gewichtsverhältnis von 0,01 bis 5,0 vorliegt.

14. Verfahren nach einem der vorhergehenden Ansprüche, wobei zumindest das erste oder das zweite Reagenz ferner ein Netzmittel, einen Entschäumer oder eine Kombination davon umfasst.

15. Verfahren nach einem der vorhergehenden Ansprüche, wobei bei Mischung der gleichen Flüssigkeitsanteile des ersten und zweiten Reagenz das resultierende Reagenzgemisch einen pH-Wert im Bereich von 4,5 bis 6,5 aufweist.

16. Verfahren nach einem der vorhergehenden Ansprüche, wobei die Pumpengeschwindigkeiten und die inneren Abmessungen der Reaktionskammer so gewählt sind, dass die vom Reagenzgemisch benötigte Zeit, um vom proximalen Ende zum distalen Ende der Reaktionskammer zu fließen, im Bereich von 20 bis 40 Sekunden liegt.

17. Verfahren nach einem der vorhergehenden Ansprüche, wobei das Druckregelventil einen Hinterdruck im Bereich von 300 bis 500 kPa erzeugt.

18. Verfahren nach einem der vorhergehenden Ansprüche, wobei die Flüssigkeitsanteile des ersten Reagenz und des zweiten Reagenz gleichzeitig zur Reaktionskammer gepumpt werden.

19. Verfahren nach einem der vorhergehenden Ansprüche, wobei der Wasserstrom durch ein Druckregelventil geführt wird, um sicherzustellen, dass das Wasser den gewünschten Mindestdruck aufweist.

20. Gerät zur Erzeugung von wässrigem Chlordioxid durch Reaktion eines ersten Reagenz und eines zweiten Reagenz, wobei das wässrige Chlordioxid verdünnt werden kann, um eine Sterilisationsflüssigkeit zur Verwendung bei der Sterilisation von medizinischen oder zahnärztlichen Geräten oder zur Wundspülung oder aseptischen Hautbehandlung zu bilden, wobei das Gerät Folgendes umfasst:
eine erste Pumpe zum Anschluss an eine Flüssigkeitsquelle des ersten Reagenz;
eine zweite Pumpe zum Anschluss an eine Flüssigkeitsquelle des zweiten Reagenz;
eine längliche Reaktionskammer mit einem proximalen und einem distalen Ende;
wobei beide Pumpen Ausgänge aufweisen, die mit dem proximalen Ende in Fluidverbindung stehen;
wobei durch Bedienung der Pumpen nach Anschluss an die Flüssigkeitsquellen Anteile des ersten und des zweiten Reagenz zunächst dem proximalen Ende der länglichen Reaktionskammer zugeführt werden, um ein Reaktionsgemisch zu bilden, und wobei durch fortgesetzten Betrieb der Pumpen das Reaktionsgemisch progressiv durch die Reaktionskammer zum distalen Ende gepumpt wird und das Reaktionsgemisch dann aus dem distalen Ende ausgestoßen wird;
wobei jede Pumpe eine Membranpumpe oder eine Kolbenpumpe ist und mit einem Druckregelventil im Flüssigkeitspfad zwischen der Pumpe und der Reaktionskammer ausgestattet ist, so dass jeder Flüssigkeitsanteil im Wesentlichen mit konstantem Druck gepumpt wird.

21. Gerät nach Anspruch 20, ferner umfassend eine mit einer Quelle fließenden Wassers verbundene Leitung, wobei die Leitung zur Aufnahme von Reaktionsgemisch aus der Reaktionskammer zur Bildung eines Stroms wässriger Chlordioxid-Sterilisationsflüssigkeit zur Verwendung bei der Sterilisation von medizinischen Geräten oder zur Wundspülung mit dem distalen Ende der Reaktionskammer in Fluidverbindung steht.

22. Gerät nach Anspruch 20 oder Anspruch 21, ferner umfassend einen ersten Behälter, der eine Flüssigkeit mit dem ersten Reagenz enthält, und einen zweiten Behälter, der eine Flüssigkeit mit dem zweiten Reagenz enthält; worin der erste Behälter mit einem Eingang der ersten Pumpe in Fluidverbindung steht und worin der zweite Behälter mit einem Eingang der zweiten Pumpe in Fluidverbindung steht.

23. Gerät nach einem der Ansprüche 20-22, wobei jede Pumpe mit einem Entlüftungsventil zum Primen der Pumpe ausgestattet ist.

24. Gerät nach einem der Ansprüche 20-23, wobei jedes Druckregelventil einen Hinterdruck im Bereich von 300 bis 500 kPa erzeugt.

25. Gerät nach einem der Ansprüche 20-24, wobei die Pumpen mit Schrittmotoren zur Steuerung des Betriebs der Pumpen ausgestattet sind.

26. Gerät nach Anspruch 25, wobei der Schrittmotorschaft jeder Pumpe mit einem optischen Codierer zur Verwendung in einer geschlossenen Feedback-Regelungsanordnung ausgestattet ist.

27. Gerät nach Anspruch 21, wobei die Leitung mit einem ersten Strömungsmesser zum Messen der Strömungsgeschwindigkeit des Wassers aus der Quelle ausgestattet ist; wobei das Gerät ferner Mittel zur Berechnung der erforderlichen ClO₂-Menge, die dem Strom zugefügt werden muss, um eine erwünschte ClO₂-Konzentration in der Sterilisationsflüssigkeit bei der gemessenen Strömungsgeschwindigkeit zu erhalten, und Mittel zum Einstellen der Pumpengeschwindigkeiten, falls erforderlich, um die gewünschte Konzentration zu erhalten, umfasst.

28. Gerät nach Anspruch 21, wobei die Leitung stromabwärts vom distalen Ende der Reaktionskammer mit einer Sonde zur Messung der ClO₂-Konzentration ausgestattet ist; wobei das Gerät ferner Mittel zum Einstellen der Pumpengeschwindigkeiten, falls erforderlich, umfasst, so dass die gemessene ClO₂-Konzentration in einen gewünschten Bereich fällt.

29. Gerät nach Anspruch 27 oder Anspruch 28, ferner umfassend Mittel zum Auslösen eines sichtbaren oder hörbaren Signals, um anzuzeigen, ob die Sterilisationsflüssigkeit innerhalb eines gewünschten Konzentrationsbereichs liegt.

30. Gerät nach Anspruch 27, wobei die Leitung mit einem zweiten, mit dem ersten Strömungsmesser in Reihe geschalteten Strömungsmesser ausgestattet ist; und wobei das Gerät ferner Mittel zum Vergleichen der Ausgabe aus beiden Strömungsmessern umfasst, um einen Vergleichswert zu erzeugen und einen Alarmzustand auszulösen, wenn der Vergleichswert einen vorbestimmten Wert überschreitet.

31. Gerät nach einem der Ansprüche 20-30, wobei die längliche Reaktionskammer eine spiralförmige Spule ist.

32. Gerät nach einem der Ansprüche 20-31, ferner umfassend ein ventilgesteuertes Mittel zum Einführen einer Durchspülflüssigkeit in die Reaktionskammer und zum Ablassen der Flüssigkeit aus der Reaktionskammer.

33. Gerät nach Anspruch 21, ferner umfassend eine ventilgesteuerte Flüssigkeitsverbindung zwischen der Leitung stromaufwärts vom distalen Ende der Reaktionskammer und dem proximalen Ende der Reaktionskammer zum Zuführen von Durchspülwasser in die Reaktionskammer.

34. Gerät nach Anspruch 33, ferner umfassend eine ventilgesteuerte Drainageverbindung aus der Reaktionskammer am oder neben dem distalen Ende zum Zuführen von Flüssigkeit aus der Reaktionskammer zu einem Abfluss.

35. Gerät nach Anspruch 28, wobei sich die Sonde in einem Reservoir befindet, das so angeordnet und angepasst ist, dass es einen Probenteil der Sterilisationsflüssigkeit, die durch die Leitung läuft, kontinuierlich auffängt.

36. Gerät nach Anspruch 20, ferner umfassend einen Sensor zur Messung der Chlordioxid-Konzentration in einem Sensorgehäuse, Mittel zur selektiven Führung von Wasser in das Sensorgehäuse, damit der Sensor feucht bleibt, Mittel zum Ablassen von Flüssigkeiten aus dem Sensorgehäuse und Mittel zum selektiven Führen des wässrigen Chlordioxids in das Sensorgehäuse, damit die Konzentration von Chlordioxid gemessen werden kann.

## Revendications

1. Procédé de production d'un courant de fluide stérilisant de dioxyde de chlore aqueux adapté pour utilisation dans la stérilisation d'équipement médical et dentaire ou pour l'irrigation de plaie ou l'asepsie de la peau, le procédé comprenant les étapes consistant à :
pomper des portions de fluide d'un premier réactif et d'un second réactif dans l'extrémité proximale d'une chambre de réaction allongée pour former un mélange de réaction, les réactifs étant choisis de manière à réagir conjointement pour former du dioxyde de chlore aqueux ;
produire un courant d'eau autour de ou adjacent à l'extrémité distale de la chambre de réaction ;
continuer de pomper des portions de fluide desdits réactifs de manière à amener le mélange de réactifs à se déplacer à travers la chambre de réaction et ensuite dans le courant d'eau pour former un courant de fluide stérilisant de dioxyde de chlore aqueux ;
dans lequel les débits de pompage et les dimensions internes de la chambre de réaction allongée sont choisis de sorte que la réaction pour former du dioxyde de chlore soit sensiblement complète lorsque le mélange de réactifs sort de la chambre de réaction ; et
continuer de pomper des portions de fluide desdits réactifs jusqu'à ce qu'une quantité souhaitée dudit fluide stérilisant ait été produite ;
dans lequel le pompage de chaque réactif est effectué au moyen d'une pompe à membrane ou d'une pompe à piston par l'intermédiaire d'une vanne de régulation de pression de sorte que chaque portion de fluide soit pompée à une pression sensiblement constante.

2. Procédé selon la revendication 1, dans lequel les pompes sont actionnées sous la commande d'un moteur pas-à-pas.

3. Procédé selon la revendication 2, dans lequel l'arbre du moteur pas-à-pas pour chaque pompe est pourvu d'un codeur optique pour utilisation dans un agencement de commande à rétroaction à boucle fermée.

4. Procédé selon l'une quelconque des revendications précédentes, comprenant en outre l'étape consistant à mesurer le débit du courant d'eau, calculer la quantité de ClO₂ devant être ajoutée au courant pour produire une concentration souhaitée de ClO₂ dans le fluide stérilisant, et ajuster les débits de pompage si nécessaire pour obtenir ladite concentration souhaitée.

5. Procédé selon l'une quelconque des revendications 1 à 3, comprenant en outre les étapes consistant à mesurer la concentration de ClO₂ dans le fluide stérilisant, comparer la concentration mesurée à une concentration souhaitée, et ajuster les débits de pompage si nécessaire pour obtenir ladite concentration souhaitée.

6. Procédé selon la revendication 4 ou revendication 5, comprenant en outre l'étape consistant à activer un signal visible ou audible pour indiquer si le fluide stérilisant est dans une plage de concentration souhaitée.

7. Procédé selon la revendication 4, dans lequel l'étape consistant à mesurer le débit comprend la mesure d'un premier débit à un emplacement en amont et un second débit à un emplacement en aval dans le courant d'eau, et le procédé comprend en outre l'activation d'un état d'alarme dans le cas où les premier et second débits diffèrent de plus d'une valeur prédéterminée.

8. Procédé selon l'une quelconque des revendications précédentes, dans lequel la chambre de réaction allongée est un enroulement hélicoïdal.

9. Procédé selon l'une quelconque des revendications précédentes, dans lequel le premier réactif est une solution aqueuse d'un chlorite, et le second réactif est une solution acide aqueuse.

10. Procédé selon la revendication 9, dans lequel le premier réactif comprend une solution aqueuse de chlorite et le second réactif comprend de l'acide citrique, de l'acide sorbique et de l'acide borique.

11. Procédé selon la revendication 10, dans lequel le second réactif comprend en outre un inhibiteur de corrosion du cuivre et du laiton, un inhibiteur de corrosion de l'acier et de l'aluminium, et un agent tampon.

12. Procédé selon la revendication 11, dans lequel l'inhibiteur de corrosion du cuivre et du laiton est un triazole ou un benzotriazole présent en un pourcentage en poids de 0,01 à 2,0.

13. Procédé selon la revendication 11 ou la revendication 12, dans lequel l'inhibiteur de corrosion de l'acier et de l'aluminium est choisi dans le groupe constitué de phosphates, de molybdates et de nitrates, et est présent en un pourcentage en poids de 0,01 à 5,0.

14. Procédé selon l'une quelconque des revendications précédentes, dans lequel au moins un des premier et second réactifs comprend en outre un agent mouillant, un agent antimousse ou une combinaison de ceux-ci.

15. Procédé selon l'une quelconque des revendications précédentes, dans lequel lorsque des portions de fluide égales des premier et second réactifs sont mélangées le mélange de réactifs résultant a un pH dans la plage de 4,5 à 6,5.

16. Procédé selon l'une quelconque des revendications précédentes, dans lequel le débit de pompage et les dimensions internes de la chambre de réaction sont choisis de sorte que le temps nécessaire pour que le mélange de réactifs se déplace de l'extrémité proximale à l'extrémité distale de la chambre de réaction est dans la plage de 20 à 40 secondes.

17. Procédé selon l'une quelconque des revendications précédentes, dans lequel la vanne de régulation de pression produit une contre-pression dans la plage de 300 à 500 kPa.

18. Procédé selon l'une quelconque des revendications précédentes, dans lequel des portions de fluide dudit premier réactif et dudit second réactif sont pompées simultanément vers ladite chambre de réaction.

19. Procédé selon l'une quelconque des revendications précédentes, dans lequel ledit courant d'eau est dirigé à travers une vanne de régulation de pression pour assurer que l'eau est à une pression minimale souhaitée.

20. Appareil pour produire du dioxyde de chlore aqueux par réaction d'un premier réactif et d'un second réactif, ledit dioxyde de chlore aqueux étant apte à la dilution pour former un fluide stérilisant adapté pour utilisation dans la stérilisation d'équipement médical et dentaire ou pour l'irrigation de plaie ou l'asepsie de la peau, l'appareil comprenant :
une première pompe pour raccordement à une source de fluide dudit premier réactif ;
une seconde pompe pour raccordement à une source de fluide dudit second réactif ;
une chambre de réaction allongée ayant des extrémités proximale et distale ;
les deux pompes ayant des sorties en raccordement fluidique avec ladite extrémité proximale ;
dans lequel le fonctionnement des pompes lorsqu'elles sont raccordées aux sources de fluide alimente des portions des premier et second réactifs initialement à l'extrémité proximale de la chambre de réaction allongée pour former un mélange de réaction et dans lequel le fonctionnement prolongé des pompes pompe progressivement le mélange de réaction à travers la chambre de réaction jusqu'à l'extrémité distale et ensuite éjecte le mélange de réaction par l'extrémité distale ;
dans lequel chaque pompe est une pompe à membrane ou une pompe à piston et est pourvue d'une vanne de régulation de pression dans le chemin de fluide entre la pompe et la chambre de réaction de sorte que chaque portion de fluide soit pompée à une pression sensiblement constante.

21. Appareil selon la revendication 20, comprenant en outre une conduite raccordée à une source d'écoulement d'eau, la conduite étant en raccordement fluidique avec l'extrémité distale de la chambre de réaction pour recevoir un mélange de réaction depuis celle-ci pour former un courant de fluide stérilisant de dioxyde de chlore aqueux adapté pour utilisation dans la stérilisation d'équipement médical ou l'irrigation de plaie.

22. Appareil selon la revendication 20 ou la revendication 21, comprenant en outre un premier récipient qui contient un fluide comprenant ledit premier réactif et un second récipient contenant un fluide comprenant ledit second réactif ; dans lequel ledit premier récipient est en raccordement fluidique avec une entrée de la première pompe et dans lequel ledit second récipient est en raccordement fluidique avec une entrée de la seconde pompe.

23. Appareil selon l'une quelconque des revendications 20 à 22, dans lequel chaque pompe est pourvue d'une vanne d'évent pour l'amorçage de la pompe.

24. Appareil selon l'une quelconque des revendications 20 à 23 dans lequel chaque vanne de régulation de pression produit une contre-pression dans la plage de 300 à 500 kPa.

25. Appareil selon l'une quelconque des revendications 20 à 24, dans lequel les pompes sont pourvues de moteurs pas-à-pas pour commander le fonctionnement des pompes.

26. Appareil selon la revendication 25, dans lequel l'arbre du moteur pas-à-pas pour chaque pompe est pourvu d'un codeur optique pour utilisation dans un agencement de commande à rétroaction à boucle fermée.

27. Appareil selon la revendication 21, dans lequel la conduite est pourvue d'un premier débitmètre pour mesurer le débit d'eau provenant de la source ; l'appareil comprenant en outre des moyens pour calculer la quantité de ClO₂ devant être ajoutée au courant pour produire une concentration souhaitée de ClO₂ dans le fluide stérilisant au débit mesuré, et des moyens pour ajuster les débits de pompage si nécessaire pour obtenir ladite concentration souhaitée.

28. Appareil selon la revendication 21, dans lequel la conduite est pourvue en aval de l'extrémité distale de la chambre de réaction d'une sonde pour mesurer la concentration de ClO₂ ; l'appareil comprenant en outre des moyens pour ajuster les débits de pompage si nécessaire de telle manière que la concentration de ClO₂ mesurée soit située dans une plage souhaitée.

29. Appareil selon la revendication 27 ou la revendication 28, comprenant en outre des moyens pour activer un signal visible ou audible pour indiquer si le fluide stérilisant est dans une plage de concentration souhaitée.

30. Appareil selon la revendication 27, dans lequel la conduite est pourvue d'un second débitmètre en série avec le premier débitmètre ; et dans lequel l'appareil comprend en outre des moyens pour comparer la sortie des deux débitmètres pour produire une valeur de comparaison et pour activer un état d'alarme dans le cas où la valeur de comparaison dépasse une valeur prédéterminée.

31. Appareil selon l'une quelconque des revendications 20 à 30, dans lequel la chambre de réaction allongée est un enroulement hélicoïdal.

32. Appareil selon l'une quelconque des revendications 20 à 31, comprenant en outre des moyens commandés par vanne pour introduire un fluide de purge dans la chambre de réaction et pour drainer le fluide depuis la chambre de réaction.

33. Appareil selon la revendication 21, comprenant en outre un raccordement de fluide commandé par vanne entre la conduite en amont de l'extrémité distale de la chambre de réaction, et l'extrémité proximale de la chambre de réaction pour alimenter de l'eau de purge dans la chambre de réaction.

34. Appareil selon la revendication 33, comprenant en outre un raccordement de drainage commandé par vanne de la chambre de réaction à ou adjacent à l'extrémité distale, pour alimenter un fluide provenant de la chambre de réaction vers un drain.

35. Appareil selon la revendication 28, dans lequel la sonde est située dans un réservoir agencé et adapté pour collecter en continu une partie d'échantillon du fluide stérilisant qui traverse la conduite.

36. Appareil selon la revendication 20, comprenant en outre un capteur pour mesurer la concentration de dioxyde de chlore dans un boîtier de capteur, des moyens pour diriger sélectivement de l'eau dans le boîtier de capteur pour maintenir le capteur mouillé, des moyens pour drainer les liquides provenant du boîtier de capteur, et des moyens pour diriger sélectivement ledit dioxyde de chlore aqueux dans ledit boîtier de capteur pour permettre que la concentration de dioxyde de chlore soit mesurée.
